# EUROPEAN PATENT APPLICATION

(11) **EP 4 811 752 A1**
(43) Date of publication of application: **23.09.2026**
(21) Application number: 26166578.0
(22) Date of filing: 20.03.2026
(51) Int. Cl.: H04L 67/125, A61B 5/00, A61C 9/00

(54) **INTRAORAL SCANNING SYSTEM WITH IMPROVED WIRELESS COMMUNICATION SECURITY**

(30) Priority: 21.03.2025 DK PA202570039
(71) Applicant: 3Shape A/S, 1060 Copenhagen K (DK)
(72) Inventor: JELLINGGAARD, Anders Robert, 1060 Copenhagen K (DK)
(74) Representative: Zacco Denmark A/S

(57) **Abstract**

According to an embodiment, an intraoral scanning system is disclosed. The intraoral scanning system may comprise an intraoral scanning device configured to acquire light information reflected from a dental object during a scanning session, one or more processors operably connected to the intraoral scanning device, and at least one of the one or more processors includes a client device and one or more servers. The one or more processors are configured to determine surface information from the light information, and generate a three-dimensional (3D) surface model of the dental object using the surface information, a wireless communication interface configured to transmit via a first wireless communication protocol a unique identifier of the intraoral scanning device, and wherein the client device is configured to receive the unique identifier via the first wireless communication protocol, and a wireless connection is established between the client device and the intraoral scanning device via the one or more servers upon a confirmation of the unique identifier by the client device.

## Description

### FIELD

The disclosure relates in general to the establishing of a wireless connection between an intraoral scanning device and a client device, and in particular to establishing a wireless connection with improved security.

### BACKGROUND

Intraoral scanning provides useful information about the characteristics of a dental object under examination. Intraoral scanning may be performed using an intraoral scanning device, that may be handheld, and includes a processing unit for obtaining scan data from the dental object. The intraoral scanning device may be connected to a display for displaying information relating to the scanned dental object, such as visualizing a digital representation of a scanned tooth. The intraoral scanning device may further be connected to a computer providing more resources for processing the obtained scan data and/or performing the displaying of information on the display, such as generating a 3D model of the scanned dental object.

Intraoral scanning systems generally include an intraoral scanning device and a client device connected to the intraoral scanning device via a wireless connection.

The intraoral scanning systems are expensive and take up space in a dental office. Accordingly, it is often unfeasible for dentist offices to invest in multiple intraoral scanning systems. This limits the number of intraoral scans that a dental office can perform. Additionally, the dentist needs to move around the entire intraoral scanning system to different rooms or needs to dedicate a single room for intraoral scanning.

The client device may be located locally and in the vicinity of the intraoral scanning device, such as in the same room, but may, in another configuration, also be located elsewhere remotely on a remote computer. The intraoral scanning device may comprise a wireless communication interface and connected to the client computer via a wireless connection. A such configuration where the intraoral scanning device, via a wireless connection, is connected to a client device, may be described as an intraoral scanning system where at least a part of the data processing is performed on a server or a cloud server. In such a configuration there is a need of improving the security in the wireless connection as more data and more sensitive data are being transferred between the devices in such a system.

### SUMMARY

An aspect of the present disclosure is to provide an easier way of establishing a wireless connection between a client device and an intraoral scanning device in a secured way.

According to the aspect, an intraoral scanning system is provided. The intraoral scanning system may include an intraoral scanning device configured to acquire light information reflected from a dental object during a scanning session. The system may further include one or more processors operably connected to the intraoral scanning device, and at least one of the one or more processors includes a client device and one or more servers.

The intraoral scanning device may be defined as a scanning device that may employ a scanning principle such as triangulation-based scanning, confocal scanning, focus scanning, ultrasound scanning, x-ray scanning, stereo vision, structure from motion, optical coherent tomography OCT, or any other scanning principle. In an embodiment, the scanning device is capable of obtaining surface information by operated by projecting a pattern and translating a focus plane along an optical axis of the scanning device and capturing a plurality of 2D images at different focus plane positions such that each series of captured 2D images corresponding to each focus plane forms a stack of 2D images. The acquired 2D images are also referred to herein as raw 2D images, wherein raw in this context means that the images have not been subject to image processing. The focus plane position is preferably shifted along the optical axis of the scanning system, such that 2D images captured at a number of focus plane positions along the optical axis form said stack of 2D images (also referred to herein as a sub-scan) for a given view of the object, i.e., for a given arrangement of the scanning system relative to the object. After moving the scanning device relative to the object or imaging the object at a different view, a new stack of 2D images for that view may be captured. The focus plane position may be varied by means of at least one focus element, e.g., a moving focus lens. The scanning device is generally moved and angled relative to the dentition during a scanning session, such that at least some sets of sub-scans overlap at least partially, in order to enable reconstruction of the digital dental 3D model by stitching overlapping subscans together in real-time and display the progress of the virtual 3D model on a display as feedback to the user. The result of stitching is the digital 3D representation of a surface larger than that which can be captured by a single sub-scan, i.e., which is larger than the field of view of the 3D scanning device. Stitching, also known as registration and fusion, works by identifying overlapping regions of 3D surface in various sub-scans and transforming sub-scans to a common coordinate system such that the overlapping regions match, finally yielding the digital 3D model. An Iterative Closest Point (ICP) algorithm may be used for this purpose. Another example of a scanning device is a triangulation scanner, where a time varying pattern is projected onto the dental object and a sequence of images of the different pattern configurations are acquired by one or more cameras located at an angle relative to the projector unit.

The one or more servers may be a combination of one or more cloud servers and/or one or more local servers. Preferably, the one or more servers is one or more cloud servers as that would not place any needs on to the user to have the needed space for the one or more servers in the clinic.

The client device may be a local computer that is used by a user to communicate with the intraoral scanning device. The client device may be a laptop, a stationary computer, a smartphone, a tablet or any portable device.

The client device may include a displaying unit, or, the client device may be connected to a displaying unit, such as a monitor.

The light information may include different wavelengths, such as visible wavelengths, UV wavelengths, and/or IR/NIR wavelengths. For instance, the intraoral scanning device may include a first light source that is configured to emit visible wavelength(s) that is between 400nm and 750 nm. The intraoral scanning device may include a second light source that is configured to emit ultraviolet wavelength(s) that is between 300 nm and 400 nm. The intraoral scanning device may include a third light source that is configured to emit infrared or near-infrared wavelength(s) that is between 750 nm and 2000 nm. The intraoral scanning device may comprise the first light source, the second light source and/or the third light source.

The one or more processors may be configured to determine surface information from the light information, and generate a three-dimensional (3D) surface model of the dental object using the surface information. In this example, the light information includes visible wavelengths. In the example where the intraoral scanning device includes a plurality of light sources including the first light source, the second light source and/or the third light source, the plurality of light sources would be turned up and down in power or switched on/off sequentially in multiple time periods, the light information would then include wavelengths of a light source of the plurality of light sources that is turned on or turned up to a certain operation power level.

Furthermore, the intraoral scanning system may include a wireless communication interface configured to transmit via a first wireless communication protocol a unique identifier of the intraoral scanning device, and wherein the client device is configured to receive the unique identifier via the first wireless communication protocol. The wireless communication interface may be part of the intraoral scanning device, the client device and/or the one or more servers. The intraoral scanning device may include an antenna that is configured to communicate via the wireless communication protocol. The client device may include an antenna that is configured to communicate to or via the wireless communication protocol. The one or more servers may include an antenna that is configured to communicate with the client device and the intraoral scanning device via the wireless communication interface, i.e. via the first wireless communication protocol. In another example, the one or more servers may not include an antenna, and is configured to communicate with intraoral scanning device and the client device via a wireless router which is wired connected to the one or more servers, and this wireless router is configured to communicate with the client device and the intraoral scanning device via the first wireless communication protocol.

A wireless connection is established between the client device and the intraoral scanning device via the one or more servers upon a confirmation of the unique identifier by the client device. The first wireless communication protocol may be configured WIFI, 60-GHz wireless communication protocol, Bluetooth, Bluetooth low energy or Near-Field-Communication (NFC).

The wireless communication interface may include an antenna that is configured to transmit and receive data/information via the first wireless communication protocol. The wireless communication interface may be configured to communicate via a second wireless communication protocol that is different from the first wireless communication protocol. The second wireless communication protocol may be configured to Wireless Fidelity (WIFI), 60-GHz wireless communication protocol, Bluetooth, Bluetooth low energy or Near-Field-Communication (NFC).

In the present disclosure, it is the intraoral scanning device that broadcasts its unique identifier via the first wireless communication protocol to the client device for the purpose of telling the client device that it is available to be wireless connected. Upon a confirmation from the client device that it would like to establish the wireless connection to the intraoral scanning device, the one or more servers would immediately establish the wireless connection to the intraoral scanning device. In the other way around, where it is the client device that broadcasts a unique identifier of an intraoral scanning device which it would like to connect to, the one or more servers would then need to check whether the intraoral scanning device is available for a connection, and if yes, then the wireless connection is established. This involves an additional step in establishing a wireless connection between the client device and the intraoral scanning device when comparing to the example where it is the intraoral scanning device that broadcasts its unique identifier.

The intraoral scanning device may be configured to broadcast by the wireless communication interface a unique identifier of the intraoral scanning device via the first wireless communication protocol, and wherein the client device confirms the unique identifier, and the confirmation is forwarded to the one or more servers which then establish the wireless connection between the intraoral scanning device and the client device.

The one or more servers may be configured to receive and forward the unique identifier to the client device via the first wireless communication protocol.

When the wireless connection has established and the intraoral scanning device is not in a scanning mode, then the intraoral scanning device would start broadcasting its unique identifier via the first wireless communication protocol once again, and thereby, other client devices of the one or more processing units would be able to wireless connect to the intraoral scanning device. In yet another example, when the wireless connection has established and the intraoral scanning device is a connection mode, then the intraoral scanning device would start broadcasting its unique identifier via the first wireless communication protocol once again, and thereby, other client devices of the one or more processing units would be able to wireless connect to the intraoral scanning device. The connection mode may be initiated by a button press or a combination of button presses on the intraoral scanning device. The connection mode may be initiated by a button press on the client device disconnecting the client device from the intraoral scanning device.

The confirmation of the unique identifier may be determined upon a user input, and the user input is a selection of the identifier device. The user input may be provided by a graphical user interface operably in connection with the client device in which a user selects the unique identifier that corresponds to the intraoral scanning device. In another example, user input may be provided by a button press on the intraoral scanning device. In this specific example, the intraoral scanning device includes an accelerometer and/or a gyroscope configured to monitor the movement of the intraoral scanning device, and the intraoral scanning device is used as an external controller of a cursor in the graphical user interface. When the user moves the intraoral scanning device the accelerometer and/or gyroscope monitors the movements and transfer the corresponding movements to the cursor, and by the button press, the user is able to select the unique identifier on the graphical user interface. In yet another example, when the client device receives the unique identifier, the one or more processors is configured to convert the unique identifier to a unique quick response code, which can then be scanned by the intraoral scanning device. The scanning of the unique quick response code displayed on the graphical user interface is seen as the confirmation of the unique identifier. The client device may receive a plurality of unique identifiers from different intraoral scanning devices, and in such an example, the one or more processors is configured to display the different unique quick response code side-by-side on the graphical user interface or in a single window on the graphical user interface wherein the different unique quick response codes are displayed one at the time with a certain frequency that makes it possible for the intraoral scanning device to scan each of the different unique quick response code in such a way that a confirmation can be provided by the client device. In the examples where the intraoral scanning device scans the unique quick response code, it is understood that it is still the client device that does the confirmation but the intraoral scanning device is seen as a device that is equivalent to a controller that selects the unique quick response code that corresponds to the intraoral scanning device in which the user wants to be connected to the client device. It is then the client device that receives a match signal from the one or more servers or the intraoral scanning device, wherein the match signal indicates whether there is a match between the displayed unique quick response code and the unique identifier of the intraoral scanning device. The match signal is then displayed on the graphical user interface which the user accepts by a use input, and thereby, the confirmation is provided by the client device, and the wireless connection is established between the client device and the intraoral scanning device via the one or more servers.

The first wireless communication protocol may be based on a wireless proximity protocol, and the confirmation of the unique identifier may be forwarded to the one or more servers, and the one or more servers may then be configured to establish the wireless connection between the client device and the intraoral scanning device via the one or more servers.

The wireless connection may include at least a second wireless communication protocol that is different from the first wireless communication protocol. In one example, the first wireless communication protocol may be used for establishing the wireless connection between the client device and the intraoral scanning device, and in the wireless connection the second wireless communication protocol may be part of. In this example, the first wireless communication protocol may be a secured wireless network protocol and the second wireless communication protocol may be an unsecured wireless network protocol. The secured wireless network protocol may include a protocol with incorporated security, such as encryption, authentication and/or integrity protection. For example, the protocol could be one of following: HTTPS, WSS (Websocket Secure), MQTTS, COAPS (Based on CoAP), SSH (Based on TCP), and QUIC (based on UDP). The incorporated security could be based on following security mechanism TLS, encryption + authentication, IPsec (ESP/AH), SRTP (Based on RTP), and/or DTLS.

The unsecured wireless network protocol may be an application level network protocol that is used in the wireless connection. Having divided the wireless connection into secured and unsecured wireless network protocols would be ideal if wanting to transmit in non-real time light information, i.e. scan data, which are not going to be displayed in real time. Then, the unsecured wireless network protocol would be ideal for light information, i.e. scan data, that are needed to be transmitted in real time and displayed in real time. For example, the surface information may be transmitted via the second wireless communication protocol to be sure that three-dimensional surface model can be rendered and displayed in real-time.

The first wireless communication protocol includes a first security level and the second wireless communication protocol includes a second security level. The first security level is different from the second security level. The first and second security level provide a level of security in the communication that affects respectively the latency in the first and second wireless communication protocol differently. The second security level may provide less latency in the communication than the first security level. The latency in communication is detrimental for the real time processing and displaying of the three-dimensional model on a displaying unit. Different types of data and/or scan data is transmitted between the intraoral scanning device and the client device and some of the scan data is relevant for the three-dimensional surface model which real time processing and displaying is detrimental for a user experience point of view, and the protocol being used for transmitting scan data for the three-dimensional model may have a lower level of security than other type of data and/or scan data (i.e. surface information) for the purpose of achieving the real time displaying/updating of the three-dimensional model on the displaying unit.

The first and second security levels may correspond to a secured and unsecured wireless network protocol, respectively.

The quality of the connection (i.e. connection quality) between the intraoral scanning device and the client device may change during use of the intraoral scanning device or due to change in location of the intraoral scanning device or the client device. Due to the change in connection quality the level of security in the first security level and/or the second security level may change to different levels by adding or removing steps or complexity of the secured/unsecured wireless network protocol, i.e. security layer optimization. For example, the surface information may comprise a header, a timestamp and a scan data layer. Ideally, all three layers should be encrypted but if the connection quality drops to a level where the real time communication between the intraoral scanning device and the client device starts to be affected negatively, then one or more of the three layers should be unencrypted. The scan data layer should always be encrypted. In another example the variation in the security level may be provided by changing the security layer. For example, the security layer in the secured/unsecured wireless network protocol may change between TLS handshake, session resumption, packet encryption or authentication. For example, typical latency provided by TLS handshake may be between 50 to 150 ms. Session resumption may introduce latency of less than 10 ms, packet encryption or authentication may introduce latency of less than 1 ms. Which means, if the connection quality drops the secured/unsecured wireless network protocol the one or more processors may apply changes to the security layer for example by disabling TLS handshake and instead enabling authentication.

The wireless communication interface may be configured to transmit via the first wireless communication protocol a unique identifier upon no requests to the client device or the one or more servers. The transmission of the unique identifier via the first wireless communication protocol may be done in a broadcasting manner. Broadcasting the unique identifier means that the intraoral scanning device doesn't need a tricker from a client device or any other external processor to initiate the broadcasting of the unique identifier.

The surface information may be transmitted, via the wireless connection, from the intraoral scanning device to the one or more servers, and the one or more servers may then be configured to generate the 3D surface model of the dental object using the surface information. The 3D surface model may then be forwarded to the client device which then displays the 3D surface model in real time.

The system may comprise a displaying unit configured to be operably connected to the client device, and wherein the displaying unit is configured to display the 3D surface model.

Latency in displaying the 3D surface model in real time may occur, and in such a situation, the user is warned via the graphical user interface that latency is occurring in the displaying of the 3D surface model, and one or more instructions to reduce the latency may appear on the graphical user interface being displayed by the displaying unit/client device. Instead of displaying one or more instructions, one or more improvement information to reduce the latency is displayed as an information to the user of which improvements that have been performed. The one or more improvement information may be to lower the resolution of the 3D surface model by lowering the amount of information to be transmitted via the wireless connection.

The first wireless communication protocol may be configured to Wireless Fidelity (WIFI), 60-GHz wireless communication protocol, Bluetooth, Bluetooth low energy or Near-Field-Communication (NFC). Which means the wireless connection may provide wireless communication via the mentioned protocols. In another example, the wireless connection may include the second wireless connection communication which may be configured to WIFI or 60-GHz wireless communication protocol, which means the wireless connection between the client device and the intraoral scanning device may include different protocols, such as WIFI and Bluetooth. Which of the first or second wireless communication protocol is used for communicating data or information depends on the type of the data or information.

The unique identifier may be forwarded directly to the client device based on proximity detection performed by the client device.

The intraoral scanning device may transmit via the first wireless communication protocol proximity data that includes the unique identifier, and wherein the client device or the one or more servers may be configured to analyse proximity data of the intraoral scanning device, (between the first client device and a user-authorized second client device;) and determine, responsive to the analysis, that a spatial proximity between the client device and the intraoral scanning device is within a threshold proximity. Furthermore, the client device and/or the one or more servers may be configured to detect, responsive to the detection of an execution of an application on the client device having the spatial proximity between the client device and the intraoral scanning device within the threshold proximity, the wireless connection between the client device and the intraoral scanning device, cause, based on the detection of the wireless connection, to visually display a wireless connection status of the wireless connection of the intraoral scanning device to the client device on a graphical user interface.

The wireless connection status includes a connection mode and an unconnected mode.

The intraoral scanning system may comprise a non-transitory memory coupled to the one or more processors. The non-transitory memory may include a set of instructions of computer-executable program code, which when executed by the one or more processors, cause the client device to concurrently detect a status of the intraoral scanning device and/or a status of the wireless connection between the client device and the intraoral scanning device.

The one or more servers may be configured to determine whether the intraoral scanning device is approved to establish the wireless connection with the client device based on the unique identifier. Then, a wireless connection prompt is generated, in response to determining that the intraoral scanning device is approved for wireless connecting with the client device, and forward the wireless connection prompt to the client device, determine, upon the client device received the wireless connection prompt, whether a wireless connection confirmation is received from the client device. Furthermore, in response to receiving the wireless connection confirmation, an intraoral scanning application query corresponding to the intraoral scanning device is generated based at least in part on the unique identifier. An intraoral scanning application server of the one or more servers is quired with an intraoral scanning device application query to retrieve an intraoral scanning application corresponding to the intraoral scanning device from the intraoral scanning application server, and establish the wireless connection between the client device and the intraoral scanning device.

The wireless connection may include a third wireless communication protocol, wherein control data of the intraoral scanning device or internal information of a dental object is transmitted via the third wireless communication protocol, and the third wireless communication protocol may be a secured wireless network protocol.

The unsecured wireless network protocol is a User Datagram Protocol (UDP) or a Transmission Control Protocol (TCP).

The client device may include an antenna, an interface circuit, electrically coupled to the antenna, configured to communicate with the one or more servers and the intraoral scanning device, wherein the interface circuit may be further configured to receive a beacon with advertising information for the intraoral scanning device, and wherein the client device may be configured to filter the advertising information, determine that the filtered advertising information has changed relative to a previous version of the advertising information for the intraoral scanning device received by the client device, and provide a wakeup signal in response to determining that the filtered advertising information has changed relative to a previous version of the advertising information, and set the client device from a power-saving mode to a normal operating mode based on the wakeup signal.

The advertising information may include the unique identifier.

The client device may comprise an intraoral scanning device certificate that may be downloaded onto the client device and stored in a memory unit of the client device. The certificate includes a second unique identifier. The confirmation provided by the client device may involve a comparing of the unique identifier and the second unique identifier, and if the two identifiers matches then the confirmation is provided and forwarded to the one or more servers. The unique identifier may be a serial number that is unique for the intraoral scanning device.

In another example, the intraoral scanning device generates a key-pair that is permanently stored in the intraoral scanning device. The intraoral scanning device transfers a public-key to one or more processors, e.g. a manufacture computer, where a certificate is generated. The certificate includes a serial number of the intraoral scanning device and the public key of the intraoral scanning device. The manufacture computer signs the certificate by using the key-pair and transmits the signed certificate to the intraoral scanning device.

The intraoral scanning device is configured to transmit the signed certificate to the client device which then uses a key-pair algorithm and a copy of the public-key which was transmitted to the client device. The client device is then configured to verify the key-pair by a key-pair algorithm and the public-key.

The intraoral scanning device may be configured to generate a public-key based on the unique identifier, and the one or more processors may be configured to receive the public key via the first wireless communication protocol, generate a certificate based on the public key, sign the certificate and transmit the signed certificate to the intraoral scanning device via the first wireless communication protocol. The intraoral scanning device may be configured to generate a signature based on the signed certificate and transmit the signature via the first wireless communication protocol to the client device, and the client device may be configured to verify the signature based on a public-key algorithm and a copy of the public-key, and the wireless connection is established when the signature is successfully verified by the client device.

The client device may include a processor of the one or more processors, a memory, coupled to the processor, which stores a program module configured to be executed by the processor. The program module including instructions for receiving the beacon, instructions for determining if the filtered advertising information is changed; and instructions for providing the wakeup signal.

The system may comprise a displaying unit configured to display a plurality of unique identifiers including the unique identifier and other unique identifiers of other intraoral scanning devices, and wherein the plurality of unique identifiers is listed according to a signal strength of a first wireless communication protocol associated to each of the plurality of unique identifiers.

The intraoral scanning device may be configured to acquire light information reflected from a three-dimensional dental object during a scanning session, wherein the scanning session is a period of time in which a scan is performed using the intraoral scanning device. The intraoral scanning device may be a handheld intraoral scanner.

The intraoral scanning system may further comprise one or more processors. The one or more processors may be operably connected to the intraoral scanning device. The one or more processors may be configured to determine, in real time, surface information from the light information, and generate a three-dimensional (3D) surface model of a dental object using the surface information. The one or more processors may comprise one processor, such as a CPU (central processing unit), with one or more processor cores. The one or more processors may comprise more than one processor, such as more a plurality of CPUs, such as a processing cluster, wherein each of the plurality of CPUs includes one or more processor cores. The intraoral scanning device and the one or more processors may be separate entities.

The intraoral scanning device and the one or more processors being separate entities may allow the processing of the data to occur outside the intraoral scanning device and may thus allow for using remote resources or may allow for a cloud-based processing, i.e. a cloud server.

The expression "remote" or "remotely" is to be understood as a location or a reference to a location, which is geographically, technically or logistically difficult to access, to reach, or to connect to, from a first location, using a wired connection, such as a data cable, or which is physically inaccessible.

Such a location may for example be a building, a city, or a country, which is located at a different location than a building, a city, or a country, than the first location, respectively.

Accordingly, the expression remote computers, remote processors, remote resources etc, are to be understood as located at remote locations as defined above, which are technically or logistically difficult to access, to reach, or to connect to, using a wired connection, such as a data cable.

For example, the intraoral scanning device, may be located at the Equator of the Earth and connected, via a wireless network of the intraoral scanning system and via the internet, to remote resources located within the polar circle of the northern hemisphere.

A such arrangement may for instance reduce the release of greenhouse gasses into the atmosphere due to the utilization of natural cooling of the remote resources, which may be computing processors that produce heat when operating, due to the proximity of the remote resources to the arctic region, and hence may reduce the contribution to increasing global warming.

Similarly, the expression "local" or "locally" is understood as being a location or a reference to a location which can be accessed, reached, or connected to, from a first location, using a wired connection, such as a data cable, or using a local wireless network, such as a wireless local area network (WLAN), such as a Wi-Fi connection to a router. Thus, in the context of this application, a local device or local resources are understood as being located in a same room in a building or in a different room in the same building. For example, the intraoral scanning device, may be located in a room, such as a dentist's clinic, and connected, via a data cable or via a wireless connection, to local resources, such as a server, a desktop computer, a laptop, a tablet, or a smartphone, located in the same room as the intraoral scanning device or located in a different room in the same building as the intraoral scanning device.

At least one of the one or more processors may be one or more remote computers.

In one aspect, where the system only comprises one processor, the processor may be located remotely relative to the intraoral scanning device, and the intraoral scanning device may be operably connected to the processor using a network connection, such as a wireless network, such as the internet, a mesh network, a short-range network, or a long-range network, such as a cellular network, i.e., 3g, 4g, 5g, or 6g. The one processor may comprise one or more processor cores. The processor may further be a computer, a tablet, a server, or more than one computer interconnected with each other. The one or more remote computers may be part of one or more groups of interconnected computing systems. The intraoral scanning device and the one or more remote computers may be separate entities.

The one or more cores or one or more processor cores may be processing units configured to read and execute program instructions.

In another aspect, where the system comprises more than one processor, the processors may be located remotely relative to the intraoral scanning device, and the intraoral scanning device may be operably connected to the processors using a network connection, such as a wireless network, such as the internet, a mesh network, a short-range network, or a long-range network, such as a telephone network, i.e., 3g, 4g, 5g, or 6g. The processors may be arranged inside one or more remote computers, one or more servers, or may be one or more remote computers or one or more servers. The processors may be several CPU's or a CPU comprising several processing cores. The intraoral scanning device and the one or more remote computers may be separate entities.

Having all the processors located remotely relative to the intraoral scanning device and outside of the intraoral scanning device may allow the intraoral scanning device to operate with a much simpler processor that allows a lower production cost of the intraoral scanning device lower heat generation, and a reduction in size and weight.

Alternatively, where the system comprises more than one processor, the processors may be distributed locally and remotely such that, for example, one processor is locally arranged inside the intraoral scanning device or inside a computer, a tablet, or a laptop, arranged in the nearby vicinity of the intraoral scanning device, such as for example in the same room, and where another processor is arranged remotely inside a remote computer operably connected to the intraoral scanning device using a wireless network, such as the internet, a mesh network, a short-range network, or a long-range network, such as a telephone network, i.e., 3g, 4g, 5g, or 6g. In a such arrangement, the remote computer may be referred to as cloud computing. The locally arranged computer may comprise a CPU comprising one or more processing cores. The remotely arranged remote computer may be one CPU comprising one or more processing cores, or may be more than one CPU each comprising one or more processing cores. The intraoral scanning device and the remote computer may be separate entities.

Having one processor arranged inside the intraoral scanning device and one processor arranged remotely outside the intraoral scanning device may allow the intraoral scanning system to distribute the processing operations between a local processor and a remote processor, or, may allow the intraoral scanning system to switch between a local processor and a remote processor. A such distribution or switching between the local processor and the remote processor may allow the intraoral scanning system, to for example, perform simpler processing operations on a local processor, and more complexed processing operations on remote processors.

The one or more remote computers may include a resource pool. The resource pool may comprise resources that are configured to process data. The resource pool may comprise resources such as hardware, or a combination of hardware and software, that are configured to process data and/or information for one or more of; scan modalities, accuracy, frame rate, colour, scan engine/reconstruction, connection/data transfer, virtual computer, user interface, and artificial intelligence/machine learning (detailed examples will be provided in the detailed description section of this disclosure). The resource pool may comprise a CPU with more than one cores, more than one CPU, and/or one or more CPU's in combination with at least one algorithm that is configured to process data. The intraoral scanning device and the resource pool may be separate entities. The resource pool may be cloud-based computing, such that the resources of the resource pool may be arranged in one or more remote computers and may be operably connected to the intraoral scanning device using a wireless network, such as the internet, a mesh network, a short-range network, or a long-range network, such as a telephone network, i.e., 3g, 4g, 5g, or 6g. The one or more remote computers may be part of the cloud-based resource pool such as a computer cluster (or just "cluster" for short) which may be collections of relatively low-cost standalone computers that are networked together. These inter-connected computers may be endowed with software to coordinate programs on (or across) those computers, and they can therefore work together to perform computationally intensive tasks.

Having a resource pool with the above mentioned resources may allow the user of the intraoral scanning system to select any of the resources or a combination of the resources, and may thereby allow an intraoral scanning device to run in different performance modes, as an on-demand solution.

The system may be configured to switch between one or more resources from the resource pool and one or more local resources of the intraoral scanning device.

A such switching between local resources and remote resources may allow the intraoral scanning system to for example perform simpler processing operations on a local processor, and perform more complexed processing operations on remote processors.

The system, via a network of the system, may further provide temporary access to the intraoral scanning device to a set of resources from the resource pool based on a predefined performance mode of the scanning session. The set of resources may comprise a combination of hardware resources, a combination of software resources, or a combination of hardware and software resources.

The hardware resource may comprise processors such as CPUs, wherein each of the CPUs may have one or more processing cores, and/or may be graphics processing units (GPUs). Software resources may be one or more algorithms, or instructions to perform computational operations, in a computer readable medium that is configured to process data.

The system may further provide temporary access for the intraoral scanning device to one or more resources of the resource pool based on a predefined performance mode of the scanning session. Further, the one or more remote computers, via a network of the system, may provide temporary access for the intraoral scanning device to the one or more resources of the resource pool based on the predefined performance mode of the scanning session. The temporary access to the one or more resources may be provided before, during or after the scanning session, depending on the selected one or more resources.

The predefined performance mode may be determined based on a user input, a user profile, or a client profile.

Basing the predefined performance mode on a user input, a user profile, or a client profile, allows the intraoral scanning system to identify the selected predefined performance mode using different selection options indicative of the desired predefined performance mode. The above will be explained further using examples in later sections.

The predefined performance mode may be instructions in a computer readable medium provided in the intraoral scanning device, in a client device connected to the intraoral scanning device, such as a computer, a tablet, or a smartphone, in one or more of the one or more remote computers, or elsewhere in the intraoral scanning system, and may be configured to be executed by a processing unit, such as a CPU, in the intraoral scanning device, in the client device connected to the intraoral scanning device, in one of the one or more remote computers, or elsewhere in the intraoral scanning system.

The predefined performance mode may be configured to operate the intraoral scanning device using;
one or more user selected resources of the resource pool, wherein the resource pool includes similar or different type of resources,,
a set of resources from at least two sets of resources from the resources pool, each set comprising a different combination of number of resources and/or different type of resources of the resource pool, or
a predefined number and/or type of resources of the resource pool.

The predefined performance mode may be one of at least two predefined performance modes that may be selected amongst several modes of operating the intraoral scanning device, such as a low-end, a mid-range, a high-end, a premium, a user defined, or a default mode of operation.

In the present disclosure, the expressions "low-end", "mid-range", "high-end", and "premium", are understood as referring to modes of operating the intraoral scanning device with certain technical specs or a certain amount and/or type of processing performance.

It is understood, that an intraoral scanning devise operating in a low-end performance mode is operating with lower technical specs, or has access to a lower amount and/or type of resources, than when operating in a medium-range performance mode, which in turn operates with lower technical specs, or has access to a lower amount and/or type of resources, than when operating in a high-end performance mode, which in turn operates with lower technical specs, or has access to a lower amount and/or type of resources, than when operating in a premium performance mode. The expression "technical specs" is to be understood as resources such as processing resources, computational processing, or other performance parameters for data handling, analysis, or processing, such as software or algorithms. The user defined mode of operation may allow a user to select individual resources, while the default mode of operation may be pre-set, such that, for example if no predefined performance mode is selected, the intraoral scanning device will operate in the default mode.

The processing performance may correspond to the processing performance of low-cost computers, average-cost computers, expensive computers, and premium computers, respectively.

In this regard, low-cost computers may be computers that are not suitable for performing complex processing tasks, but may be suitable for word processing or internet browsing. Average-cost computers may be computers that are further suitable for performing more complex processing tasks, such as running simple games and video streaming. Expensive computers may be computers that are yet further suitable for performing complex processing such as running complex games and CAD software. Premium computers may be computers that are yet further suitable for performing more complex processing such as handling a large amount of data or video editing.

For example, when a low-end performance mode of operating the intraoral scanning device is selected, the intraoral scanning device may only have access to one computational processing unit (CPU). In another example, when a medium-range performance mode is selected, the intraoral scanning device may have access to two CPUs. In yet another example, when a high-end performance mode is selected, the intraoral scanning device may have access to two CPUs and to an algorithm for generating a 3D model. In a further example, when a premium performance mode is selected, the intraoral scanning device may have access to two CPUs, an algorithm for generating a 3D model, a graphics processing unit (GPU), and Artificial Intelligence.

The predefined performance mode may be one of at least two predefined performance modes. The predefined performance mode may be one of several predefined modes, such as a low-end, a mid-range, a high-end, a premium, a default, and a user defined performance mode.

Each of the at least two predefined performance modes may be configured to operate the intraoral scanning device with a different number of resources from the resource pool and/or with a different type of resources from the resource pool.

One of the at least two predefined performance modes may comprise one of a low-end mode, a mid-range mode, a high-end mode, a premium mode, a user defined mode, or a default mode, and another one of the at least two predefined performance modes may comprise a different one of a low-end mode, a mid-range mode, a high-end mode, a premium mode, a user defined mode, or a default mode.

Having an intraoral scanning system that comprises an intraoral scanning device that is configured to be operated in more than one predefined performance modes that differ in number and/or type of accessible resources from a resource pool may allow the intraoral scanning device to run in different performance modes independent of the specifications of the scanning device at the time of purchase.

The temporary access may be achieved for example by having the system configured to disallow access of the one or more resources for the scanning session in response to the intraoral scanning device no longer utilizing the one or more resources.

Providing a temporary access for the intraoral scanning device to one or more resources of the resource pool based on a predefined performance mode may allow for an on-demand solution for operating an intraoral scanning device.

The temporary access may further be provided by a resource controller which may be an algorithm, that may be a computer readable medium, that may be provided in a software in the intraoral scanning device, in a local computer connected to the intraoral scanning device and part of the intraoral scanning system, in the one or more remote computers, in a computer connected to the one or more remote computers and part of the intraoral scanning system, or in the resource pool. The resource controller may be configured to provide access for the intraoral scanning device to the one or more resources based on a user input, a user profile, and/or a client profile.

Thus, the intraoral scanning system may further comprise a resource controller that may be configured to control the access to the one or more resources.

The access may be based on the predefined performance mode of the scanning session and may be associated with the generation of the three-dimensional (3D) surface model of the dental object using the surface information.

A resource controller may allow the intraoral scanning device to access the resources of the resource pool according to a user input, a user profile, and/or a client profile.

In a situation where the access is based on the user input, the resource controller may be configured to allow access to the one or more resources according to a selection performed by a user of the intraoral scanning device.

For example, a user, such as a dentist, may upon the initiation of a scanning session, select a performance mode for the scanning session, that will decide the number and/or type of resources that will be accessed by or allocated for the intraoral scanning device from the resource pool during the scanning session. Upon the termination of the scanning session, the resources are disallowed such that they are available for allocation or assess again. The user may for example select between two or more predefined performance modes, such as a low-end mode, a default mode, or a high-end mode, giving access to predefined number and/or type of resources of the one or more resources.

Alternatively, the user may select the desired resources, such as the number of processors, graphics quality, a frame rate, and/or to include artificial intelligence (AI).

The predefined performance mode may thus comprise one of a low-end mode, a mid-range mode, a high-end mode, a premium mode, a user defined mode, or a default mode.

The user may select the desired predefined performance mode or select the desired resources via a user interface, which may be arranged on the intraoral scanning device or on a graphical user interface on a display which may be connected to a local computer, a smartphone, or a tablet, and may be arranged nearby in the vicinity of the intraoral scanning device.

The resource controller may then allow access for the intraoral scanning device to the resources of the resource pool according to the selected predefined performance mode or the selected resources. The resource controller may allow access for example by allocating one or more resources of the resource pool according to the selected predefined performance mode or to the selected resources.

Basing the access of the intraoral scanning device to the resources of the resource pool on a user input may allow the user of the intraoral scanning device to freely select a desired number, type, set, or combination of the resources of the resource pool

In a situation, where the access is based on a user profile, the resource controller may be configured to allow access for the intraoral scanning device to a predefined number and/or type of resources, and thus operate the intraoral scanning device in a predefined performance mode.

For example the user, such as a dentist, may sign into the user's profile on a web portal, such as a website that may be connected to the intraoral scanning system, using for example personal credentials, and based on a predefined performance mode that may be associated with the user's profile, the resource controller provides access for the intraoral scanning device to a number and/or type of resources from the resource pool, that are associated to or correspond to the predefined performance mode. The predefined performance mode may be previously set as the default performance mode for the user's profile, and may differ for each user having a profile on the web portal that is associated with the intraoral scanning system. Several user profiles with different predefined performance modes may be run one at a time on the same intraoral scanning device. An intraoral scanning device may thus be operated with a low-end performance mode having access to a number and/type of resources from the resource pool for one user profile in one intraoral scanning session, and the same intraoral scanning device may be operated with a high-end performance mode having access to a larger number of resources and/or more types of resources from the resource pool, when a different user profile is signed in, in another intraoral scanning session. This might be a particular advantage in a situation where multiple users of a intraoral scanning system are performing different tasks requiring access to different resources. One example could be a denturist particularly focused on scanning edentulous patients with the aim of creating dentures and a dentist using the intraoral scanning system to perform diagnostic assessment which would require access to a different set of resources then performing edentulous scanning.

Basing the access of the intraoral scanning device to the resources of the resource pool on a user profile may allow the user of the intraoral scanning device to simply sign into the user's profile and operate the intraoral scanning device in a previously selected performance mode, without having to repeatedly select a desired number, type, set, or combination of resources, or having to repeatedly choose a performance mode, each time the user wants to use the intraoral scanning device.

In a situation where the access is based on a client profile, the resource controller may provide access for the intraoral scanning device to the resources of the resource pool according to a predefined performance mode, that may have been pre-set for the client profile. The client may be an intraoral scanning device. The client may further be a computer, a tablet, or a smartphone, that are connected to the intraoral scanning device or the intraoral scanning system, and may be located locally in the near vicinity of the intraoral scanning device, such as in a clinic of a dentist.

For example, when the intraoral scanning device is connected to the intraoral scanning system, for example using the internet, the resource controller recognizes that particular intraoral scanning devices, and provides access for that particular intraoral scanning device to a number and/or type of resources from the resource pool, that have been predefined or pre-set for it at an earlier stages, such as a default performance mode. The user of the intraoral scanning device, may via a user interface, such as buttons on the intraoral scanning, a display, or a graphical user interface, switch to another performance mode, which may be a predefined performance mode or directly select a different number and/or type of resources from the resource pool. The resource controller may then provide access for that particular intraoral scanning device to the different number and/or type of resources according to the most recently selected predefined performance mode or to the most recently selected number and/or type of resources, whenever that particular intraoral scanning device is connected to the intraoral scanning system.

Basing the access of the intraoral scanning device to the resources of the resource pool on a client profile may allow the user of the intraoral scanning device to simply connect the intraoral scanning device to the intraoral scanning system and operate the intraoral scanning device in a previously selected performance mode, without having to repeatedly sign in with a user profile, each time the user wants to use the intraoral scanning device.

The resource controller may be configured to receive a request from the intraoral scanning device or the intraoral scanning system, such as for example through a user input via a user interface or through a profile signing into a web portal or website of the intraoral scanning system, such as a user profile or client profile.

The user input may be a haptic signal through a touching of a user on a physical or digital button, or may be an input via a graphical user interface on a display connected to the intraoral scanning device or to the intraoral scanning system, such as on a software or a website, where the user input may indicate a user profile signing in.

The resource controller may further be configured to receive a request through the detection of an intraoral scanning device being connected to the intraoral scanning system. Upon receiving request, the resource controller may grant access for the intraoral scanning device to the resources or sending a signal to the resource pool to allow access.

The resource controller may further be configured to provide access for the intraoral scanning device to the resource of the resource pool based on a detection of an intraoral scanning device or a client, such as a computer, tablet, or smartphone that is connected to the intraoral scanning device, being connected to the intraoral scanning system.

The intraoral scanning device may thus be configured to access the resources of the resource pool on an on-demand basis.

The resource controller may be configured to disallow access to the one or more resources for the scanning session in response to the intraoral scanning device no longer utilizing the one or more resources.

The system or resource controller may be configured to disallow the intraoral scanning device from utilizing the one or more resources.

Having a resource controller that is configured to disallow access for the intraoral scanning device to the one or more resources of the resource pool may allow the resource controller to provide access or allocate the resources to other applications or other intraoral scanning devices that are connected to the intraoral scanning system, and may prevent having idle resources, when they may be used elsewhere.

The system may further comprise a conflict controller that may control a resource conflict between the intraoral scanning device and at least one other intraoral scanning device of the system that may compete for a same resource of the one or more resources.

The conflict controller may be an algorithm in a computer readably medium such as in a software and may be a part of the intraoral scanning system, such as in the intraoral scanning device, in a client device such as a computer, tablet, or smartphone that is connected to the intraoral scanning device, or in one or more of the one or more remote computers. The conflict controller may further be a part of the resource pool. The conflict controller may further be part of the resource manager. The conflict controller may be comprised in the resource pool or in one of the one or more remote computers.

Having a conflict controller may prevent a conflict between two or more intraoral scanning devices connected to the intraoral scanning system and competing for the same resource or the same set of resource.

The conflict controller may provide access to the one or more resources to the intraoral scanning device or to the at least one other intraoral scanning device based on a predefined performance mode for a scanning session of each of the respective intraoral scanning devices.

For example, if two intraoral scanning devices, one operating in a low-end performance mode and the other operating in a high-end performance mode, compete for the same resource, the conflict controller may be configured to allow the intraoral scanning device operating in a high-end performance mode to access that resource.

Allowing access to a resource based on a predefined performance mode may prevent a conflict between intraoral scanning devices competing for a same resource.

The conflict controller may provide access to the one or more resources based on the earliest of requests for providing access to the one or more resources or on a predetermined priority level of the scanning session. The predetermined priority level may be a ranking of a user profile or a client profile, or may be based on a user input.

The ranking may be based on a selected predetermined performance mode, a seniority level, or an urgency level.

For example, if two intraoral scanning devices send a request for accessing the same resource, the conflict controller will determine which of the two intraoral scanning device to get access to the resource based on which of the two requests has been sent or received earliest.

Allowing access to a resource based on a the earliest of requests or a predetermined priority level, may prevent a conflict between intraoral scanning devices competing for a same resource.

According to the aspect, an intraoral scanning system is provided that may comprise an intraoral scanning device that may be configured to acquire intraoral scan data from a three-dimensional dental object during a scanning session. The intraoral scanning device may comprise a processing unit that may be configured to process intraoral scan data of a patient and provide 2D image data and/or 3D image data. The intraoral scanning device may further comprise a wireless interface that may be configured to transmit the 2D image data and/or the 3D image data. The intraoral scanning device may further comprise a memory. The intraoral scanning system may further comprise one or more remote computers with a resource pool. The one or more remote computers may via a network of the system provide temporary access to the intraoral scanning device to one or more resources of the resource pool. The access for the intraoral scanning device to the one or more resources of the resource pool may be based on a predefined performance mode for the scanning session.

According to the aspect, an intraoral scanning system is provided that may comprise an intraoral scanning device that may be configured to acquire visible light information reflected from a three-dimensional dental object during a scanning session. The intraoral scanning system may further comprise one or more processors that may be operably connected to the intraoral scanning device. The one or more processors may be configured to receive visible light information from the intraoral scanning device. The intraoral scanning device may further be configured to determine, in real time, surface information from the visible light information, and may be configured to generate a three-dimensional (3D) surface model of a subject's teeth using the surface information. By determining surface information in real time, it is understood, that a time between sending the visible light information and determining surface information, which is the latency of this operation, is less than 1 second.

The intraoral scanning system may further be configured to generate the three-dimensional (3D) surface model of a subject's teeth using the surface information in real time, wherein the term "real time" in this context is to be understood as the beginning of the generation of the three-dimensional (3D) surface model, upon the receipt of the surface information. At least one of the one or more processors may be one or more remote computers that may have a resource pool. The one or more remote computers may via a network of the system, provide temporary access for the intraoral scanning device to one or more resources of the resource pool. The access may be based on a predefined performance mode of the scanning session and may be associated with the generation of the three-dimensional (3D) surface model of a subject's teeth using the surface information.

The predefined performance mode may be one of at least two predefined performance modes. Each of the at least two predefined performance modes may associate with a different number of resources from the resource pool and/or with a different type of resources from the resource pool.

Operating an intraoral scanning device using resources from a resource pool in one or more remote computers based on a predefined performance mode allows for an intraoral scanning device to be operated in different performance modes without having to select resources individually.

The one or more remote computers may further comprise a resource controller that may be configured to control an allocation of the resources.

The system may further be configured to de-allocate the resources for the scanning session in response to the intraoral scanning device no longer utilizing the one or more resources.

Having a resource controller that is configured to allocate and de-allocate resources of the resource pool to an intraoral scanning device may allow an intraoral scanning device to be operated in an on-demand basis.

In another example, the intraoral scanning system may further comprise a smartphone with a camera comprising one or more lenses or a digital camera that is configured to be connected to a wireless network and comprising one or more lenses. The intraoral scanning system may be configured to allow the smartphone or the digital camera to complement, assist, or partly substitute the intraoral scanning device, by allowing the smartphone or the digital camera to perform operations such as obtaining images or acquiring light information of the dental object.

Complementing, assisting, or partly substituting the intraoral scanning device with a smartphone or a digital camera may allow for acquiring light information using the smartphone or digital camera, and process the acquired information using the one or more resources of the resource pool. For example, a patient may use a smartphone that is connected to the intraoral scanning system to take pictures or a video of the patient's teeth, and the information may be processed by the one or more resources of the resource pool, without having to go to a dentist or to a clinic.

According to the aspect, a method for an intraoral scanning system is provided. The method may comprise the step of acquiring light information reflected from a three-dimensional dental object during a scanning session.

The acquiring may be conducted using optical equipment such as a light source, an optical lens, and an image sensor. The method may further comprise the step of determining in real time surface information from the light information via an intraoral scanning device.

The acquired light information may be processed in real time using one or more processors. The processed information may allow for visualising the dental object on a display in real time. The method may further comprise the step of generating a three-dimensional (3D) surface model of a dental object using the surface information via one or more processors. The one or more processors may be at least partly one or more remote computers having a resource pool.

The one or more remote computers may be one or several interconnected computers, or may be a server, located remotely from the intraoral scanning device, and connected to the intraoral scanning device via a wireless network, such as the internet.

The resource pool may be resources, that may be configured to process light information, an may be hardware, such as one or more CPU's, software, such as one or more algorithms, or a combination of hardware and software.

The method may further comprise the step of providing temporary access for the intraoral scanning device to one or more resources of the resource pool based on a predefined performance mode of the scanning session. The predefined performance mode may be one of at least two predefined performance modes, wherein each of the at least two predefined performance modes may be configured to operate the intraoral scanning device with a different number of resources from the resource pool and/or a different type of resources from the resource pool.

The temporary access may be achieved for example by having the system configured to disallow access of the one or more resources for the scanning session in response to the intraoral scanning device no longer utilizing the one or more resources.

The method may further comprise the step of operating the intraoral scanning device with a number of resources of the resource pool and/or a type of resources from the resource pool that may be based on the predefined performance mode.

Providing a temporary access for the intraoral scanning device to one or more resources of the resource pool based on a predefined performance mode may allow for an on-demand solution for operating an intraoral scanning device.

The method may further base the predefined performance mode on one or more of:
a user input, a user profile, and/or a client profile.

Basing the predefined performance mode on a user input, a user profile, or a client profile, allows the intraoral scanning system to identify the selected predefined performance mode using different options.

The method may further comprise the step of disallowing access for the intraoral scanning device to the one or more resources in response to an ending criteria. The ending criteria may be one or more of a measured value, a time limit, a minimum connection quality, an ending signal, or that the intraoral scanning device no longer utilizes the one or more resources.

Disallow the access for the intraoral scanning device to the one or more resources of the resource pool may allow the system to provide access or allocate the resources to other applications or other intraoral scanning devices that are connected to the intraoral scanning system, and may prevent having idle resources, when they may be used elsewhere.

The method may comprise the step of selecting the predefined performance mode that may comprise one of a low-end mode, a mid-range mode, a high-end mode, a premium mode, a user defined mode, or a default mode. The default mode may be pre-set by the manufacturer or by the user, and may be adjustable after being set.

Selecting one of the above mentioned modes may allow a user to operate an intraoral scanning device with a predefined performance mode and not having to select several resources individually.

The method may further apply for an intraoral scanning system wherein the one or more remote computers may be part of a cloud based resource pool or wherein the one or more resources may be part of one or more groups of interconnected computing systems. A such arrangement may allow the method to be applied for an intraoral scanning system wherein the resource for processing data are accessed on an on-demand basis.

The method may further comprise the step of identifying the predefined performance mode based on a user action.

The user action may be a user input using the intraoral scanning device or a display, may be connecting the intraoral scanning device to the intraoral scanning system, or may be signing in using a user profile or a client profile.

Identifying the predefined performance mode based on a user action allows the user to operate the intraoral scanning device in a predefine performance mode without having to select several processing resources individually.

Those skilled in the art will recognize still other aspects of the present application upon reading and understanding the attached description.

### BRIEF DESCRIPTION OF THE FIGURES

Aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 illustrates an example of an intraoral scanning system comprising an intraoral scanning device and one or more processors;
FIGS. 2A and 2B illustrate examples of establishment of wireless connection between a client device and an intraoral scanning device;
FIGS. 3A, 3B and 3C illustrate another example of establishment of wireless connection between a client device and an intraoral scanning device,
FIGS.4A, 4B,4C,4D illustrate examples of different wireless connections,
FIG. 5 illustrates an example of establishment of wireless connection between a client device and an intraoral scanning device,
FIG. 6 illustrates an example of establishment of wireless connection between a client device and an intraoral scanning device, and
FIG. 7 illustrates an example of powering an intraoral scanning device.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the devices, systems, mediums, programs and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A scanning for providing intra-oral scan data may be performed by a dental scanning system that may include an intraoral scanning device such as the TRIOS series scanners from 3Shape A/S or a laboratory-based scanner such as the E-series scanners from 3 Shape A/S. The dental scanning system may include a wireless capability as provided by a wireless interface such as a wireless network unit. The scanning device may employ a scanning principle such as triangulation-based scanning, confocal scanning, focus scanning, ultrasound scanning, x-ray scanning, stereo vision, structure from motion, optical coherent tomography OCT, or any other scanning principle. In an embodiment, the scanning device is capable of obtaining surface information by operated by projecting a pattern and translating a focus plane along an optical axis of the scanning device and capturing a plurality of 2D images at different focus plane positions such that each series of captured 2D images corresponding to each focus plane forms a stack of 2D images. The acquired 2D images are also referred to herein as raw 2D images, wherein raw in this context means that the images have not been subject to image processing. The focus plane position is preferably shifted along the optical axis of the scanning system, such that 2D images captured at a number of focus plane positions along the optical axis form said stack of 2D images (also referred to herein as a sub-scan) for a given view of the object, i.e., for a given arrangement of the scanning system relative to the object. After moving the scanning device relative to the object or imaging the object at a different view, a new stack of 2D images for that view may be captured. The focus plane position may be varied by means of at least one focus element, e.g., a moving focus lens. The scanning device is generally moved and angled relative to the dentition during a scanning session, such that at least some sets of sub-scans overlap at least partially, in order to enable reconstruction of the digital dental 3D model by stitching overlapping subscans together in real-time and display the progress of the virtual 3D model on a display as feedback to the user. The result of stitching is the digital 3D representation of a surface larger than that which can be captured by a single sub-scan, i.e., which is larger than the field of view of the 3D scanning device. Stitching, also known as registration and fusion, works by identifying overlapping regions of 3D surface in various sub-scans and transforming sub-scans to a common coordinate system such that the overlapping regions match, finally yielding the digital 3D model. An Iterative Closest Point (ICP) algorithm may be used for this purpose. Another example of a scanning device is a triangulation scanner, where a time varying pattern is projected onto the dental object and a sequence of images of the different pattern configurations are acquired by one or more cameras located at an angle relative to the projector unit.

Colour texture of the dental object may be acquired by illuminating the object using different monochromatic colours such as individual red, green and blue colours or my illuminating the object using multichromatic light such as white light. A 2D image may be acquired during a flash of white light.

Generally, the process of obtaining surface information in real time of a dental object to be scanned requires the scanning device to illuminate the surface and acquire high number of 2D images. Typically, a high-speed camera is used with a framerate of 300-2000 2D frames pr second dependent on the technology and 2D image resolution. The high amount of image data needed to be handled by the scanning device to either directly forward the raw image data stream to an external processing device or performing some image processing before transmitting the data to an external device or display. This process requires that multiple electronic components inside the scanner is operating with a high workload thus requiring a high demand of current.

The scanning device comprises one or more light projectors configured to generate an illumination pattern to be projected on a three-dimensional dental object during a scanning session. The light projector(s) preferably comprises a light source, a mask having a spatial pattern, and one or more lenses such as collimation lenses or projection lenses. The light source may be configured to generate light of a single wavelength or a combination of wavelengths (mono- or polychromatic). The combination of wavelengths may be produced by using a light source configured to produce light (such as white light) comprising different wavelengths. Alternatively, the light projector(s) may comprise multiple light sources such as LEDs individually producing light of different wavelengths (such as red, green, and blue) that may be combined to form light comprising the different wavelengths. Thus, the light produced by the light source may be defined by a wavelength defining a specific colour, or a range of different wavelengths defining a combination of colours such as white light. In an embodiment, the scanning device comprises a light source configured for exciting fluorescent material of the teeth to obtain fluorescence data from the dental object. Such a light source may be configured to produce a narrow range of wavelengths. In another embodiment, the light from the light source is infrared (IR) light, which is capable of penetrating dental tissue. The light projector(s) may be DLP projectors using a micro mirror array for generating a time varying pattern, or a diffractive optical element (DOF), or back-lit mask projectors, wherein the light source is placed behind a mask having a spatial pattern, whereby the light projected on the surface of the dental object is patterned. The back-lit mask projector may comprise a collimation lens for collimating the light from the light source, said collimation lens being placed between the light source and the mask. The mask may have a checkerboard pattern, such that the generated illumination pattern is a checkerboard pattern. Alternatively, the mask may feature other patterns such as lines or dots, etc.

The scanning device preferably further comprises optical components for directing the light from the light source to the surface of the dental object. The specific arrangement of the optical components depends on whether the scanning device is a focus scanning apparatus, a scanning device using triangulation, or any other type of scanning device. A focus scanning apparatus is further described in EP 2 442 720 B1 by the same applicant, which is incorporated herein in its entirety.

The light reflected from the dental object in response to the illumination of the dental object is directed, using optical components of the scanning device, towards the image sensor(s). The image sensor(s) are configured to generate a plurality of images based on the incoming light received from the illuminated dental object. The image sensor may be a high-speed image sensor such as an image sensor configured for acquiring images with exposures of less than 1/1000 second or frame rates in excess of 250 frames pr. Second (fps). As an example, the image sensor may be a rolling shutter (CCD) or global shutter sensor (CMOS). The image sensor(s) may be a monochrome sensor including a colour filter array such as a Bayer filter and/or additional filters that may be configured to substantially remove one or more colour components from the reflected light and retain only the other non-removed components prior to conversion of the reflected light into an electrical signal. For example, such additional filters may be used to remove a certain part of a white light spectrum, such as a blue component, and retain only red and green components from a signal generated in response to exciting fluorescent material of the teeth.

The wireless network unit is configured to wirelessly connect the intraoral scanning system to a network comprising a plurality of network elements including at least one network element configured to receive the processed data.

The dental scanning system preferably further comprises a processor configured to generate scan data (such as intra-oral scan data) by processing the two-dimensional (2D) images acquired by the scanning device. The processor may be part of the scanning device. As an example, the processor may comprise a Field-programmable gate array (FPGA) and/or an Advanced RISC Machines (ARM) and/or a x86 processor and/or a combination of FPGA, ARM, and/or x86 processors located on the scanning device. The scan data comprises information relating to the three-dimensional dental object. The scan data may comprise any of: 2D images, 3D point clouds, depth data, texture data, intensity data, colour data, and/or combinations thereof. As an example, the scan data may comprise one or more-point clouds, wherein each point cloud comprises a set of 3D points describing the three-dimensional dental object. As another example, the scan data may comprise images, each image comprising image data e.g., described by image coordinates and a timestamp (x, y, t), wherein depth information can be inferred from the timestamp. The image sensor(s) of the scanning device may acquire a plurality of raw 2D images of the dental object in response to illuminating said object using the one or more light projectors. The plurality of raw 2D images may also be referred to herein as a stack of 2D images. The 2D images may subsequently be provided as input to the processor, which processes the 2D images to generate scan data. The processing of the 2D images may comprise the step of determining which part of each of the 2D images are in focus in order to deduce/generate depth information from the images. The depth information may be used to generate 3D point clouds comprising a set of 3D points in space, e.g., described by cartesian coordinates (x, y, z). The 3D point clouds may be generated by the processor or by another processing unit. Each 2D/3D point may furthermore comprise a timestamp that indicates when the 2D/3D point was recorded, i.e., from which image in the stack of 2D images the point originates. The timestamp is correlated with the z-coordinate of the 3D points, i.e., the z-coordinate may be inferred from the timestamp. Accordingly, the output of the processor is the scan data, and the scan data may comprise image data and/or depth data, e.g., described by image coordinates and a timestamp (x, y, t) or alternatively described as (x, y, z). The scanning device may be configured to transmit other types of data in addition to the scan data. Examples of data include 3D information, texture information such as infra-red (IR) images, fluorescence images, reflectance colour images, x-ray images, and/or combinations thereof.

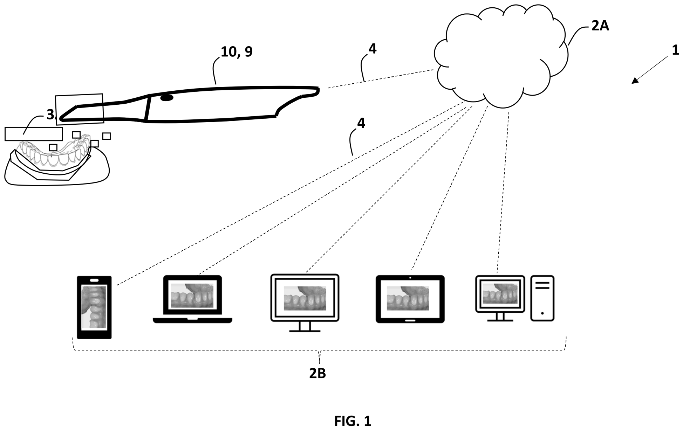

FIG.1 illustrates an intraoral scanning system that includes an intraoral scanning device 10 that is configured to acquire light information reflected from a dental object 3 during a scanning session. The system 1 includes one or more processors (2A, 2B) which in this example includes a cloud server 2A and a client device 2B. In yet another example, the intraoral scanning device may include a processor that forms part of the one or more processors. The one or more processors are configured to determine surface information from the light information, and generate a three-dimensional (3D) surface model of the dental object using the surface information. In FIG. 1, different examples of a client device 2B is seen. For example, the client device 2B could be a smartphone, a laptop, a monitor with inbuild processor, a tablet and a desktop computer. In the example illustrated in FIG. 1, a wireless connection 4 is established between the intraoral scanning device 10 and the client device 2B. The system 1 further includes a wireless communication interface 9 that forms part of the intraoral scanning device 10.

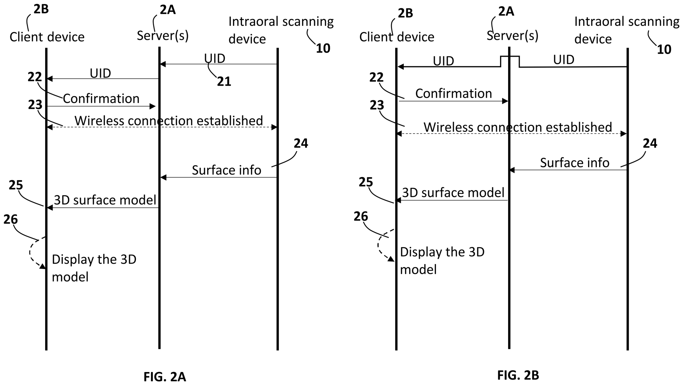

FIGS. 2A and 2B illustrate examples of establishment of the wireless connection 4. The wireless communication interface 9 which in this example forma part of the intraoral scanning device 10 transmits via a first wireless communication protocol a unique identifier 21 of the intraoral scanning device 10, and wherein the client device 2B is configured to receive the unique identifier 21 via the first wireless communication protocol and from the one or more servers 2A. A wireless connection 4 is established 23 between the client device 2B and the intraoral scanning device 10 via the one or more servers 2A upon a confirmation 22 of the unique identifier 21 by the client device 2B. After the wireless connection 23 has been established, the surface information 24 may be transmitted via the wireless connection 4 to the one or more servers which then determines a three-dimensional model 25 that is forwarded to the client device 2B and displayed 26. In FIG. 2B, the unique identifier 21 is forwarded to the client device 2B directly from the intraoral scanning device 10. In this example, the first wireless communication protocol is based on a wireless proximity protocol. In this example, the client device is configured to confirm the unique identifier automatically or manually as when the intraoral scanning device is within a proximity threshold which is evaluated based on proximity data that is transmitted by the intraoral scanning device. The proximity data does include the unique identifier. The client device 2B or the one or more servers 2A is configured to analyse the proximity data of the intraoral scanning device, determine, responsive to the analysis, that a spatial proximity between the client device and the intraoral scanning device is within a threshold proximity; detect, responsive to the detection of an execution of an application on the client device having the spatial proximity between the client device and the intraoral scanning device within the threshold proximity, the wireless connection between the client device and the intraoral scanning device; and cause, based on the detection of the wireless connection, to visually display a wireless connection status of the wireless connection of the intraoral scanning device to the client device on a graphical user interface.

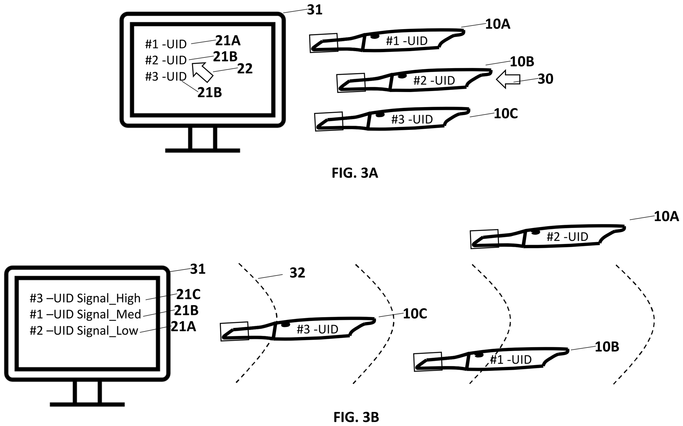

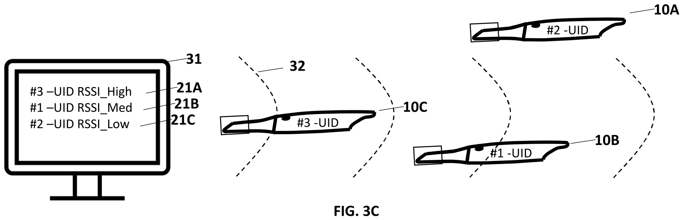

FIGS. 3A and 3B illustrate examples on the displaying of the unique identifier 21 on a displaying unit 31. In FIG. 3A, a plurality of unique identifiers (21A, 21B, 21C) of different intraoral scanning devices (10A, 10B, 10C) may appear on a list in the graphical user interface of the displaying unit 31. In FIG. 3A the plurality of unique identifiers (21A, 21B, 21C) is randomly ranked or ranked according to a timestamp of when the client device 2B received a corresponding unique identifier 21. In the example illustrated in FIG. 3A a user is using 30 the intraoral scanning device 10B, and thereby, confirms the use of the intraoral scanning device 10B by selecting the corresponding unique identifier 21B on the graphical user interface of the displaying unit 31. In FIG. 3B, the plurality of unique identifiers (21A, 21B, 21C) of corresponding intraoral scanning devices (10A, 10B, 10C) are ranked according to a signal strength of proximity data received by the client device 2B from a corresponding intraoral scanning device (10A,10B,10C). In this example, the intraoral scanning device 10C is closest to the client device 2B, and therefore, a corresponding graphical user interface to the client device 2B has ranked the unique identifier 21C of the corresponding intraoral scanning device 10C highest. In FIG. 3C, the plurality of unique identifiers are ranked according to a Received Signal Strength Indicator (RSSI) signal that represents the signal strength of signals, such as the unique identifier, received by the client device and transmitted by a corresponding intraoral scanning device (10A, 10B, 10C).

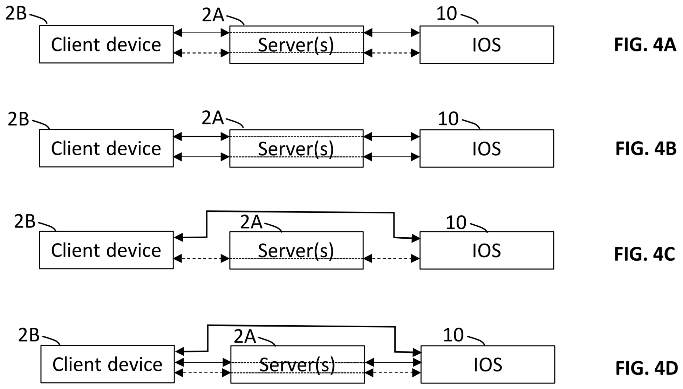

FIGS. 4A, 4B, 4C and 4D illustrate different examples of the wireless connection 4 that includes a first 41 and a second 42 wireless communication protocols. In FIG. 4A, the first wireless communication protocol 41 is a secured communication protocol and is configured to transmit the unique identifier, status information of the intraoral scanning device, diagnostic information that relates to the patient being scanned. The second wireless communication protocol 42 is an unsecured communication protocol that is configured to transmit data/information that needs real time processing of the one or more processors (2A,2B), such as surface information that needs to be real time processes such that a three-dimensional surface model can be rendered in real time on a displaying unit. In FIG. 4C, the first wireless communication protocol is bypassing the one or more server(s) 2A in order to communicate directly with the client device 2B. In such an example, the first wireless communication protocol 41 relates to a proximity protocol, such as NFC, or a Bluetooth protocol, and, wherein the second wireless communication protocol 42 relates to a protocol that may include one or more of following protocols WIFI, 60-GHz wireless communication protocol, 3G, 5G, 6G, or 7G internet connection. FIG. 4D includes a third wireless communication protocol 43 that relates to a proximity protocol, such as NFC, or a Bluetooth protocol, and, wherein the first 41 and the second 42 wireless communication protocol relate to a protocol that may include one or more of following protocols WIFI, 60-GHz wireless communication protocol, 3G, 5G, 6G, or 7G internet connection.

The first 41, second 42 and third 43 wireless communication protocol may be secured/unsecured depending on the information to be transmitted via the respective protocols.

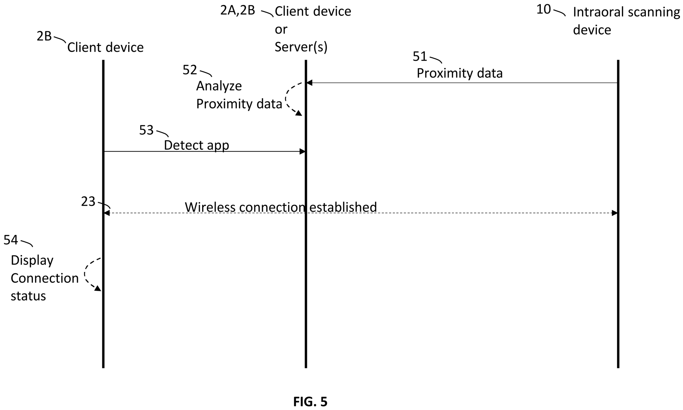

FIG. 5 illustrates an example of displaying a connection status of the intraoral scanning device 10 and the client device 2B. In this example, the intraoral scanning device 10 transmits via the first wireless communication protocol proximity data 51 that includes the unique identifier 21. The client device 2B or the one or more servers 2A is configured to analyse 52 proximity data of the intraoral scanning device, determine, responsive to the analysis, that a spatial proximity between the client device and the intraoral scanning device is within a threshold proximity. The one or more server(s) 2A is configured to detect 53, responsive to the detection of an execution of an application on the client device 2B having the spatial proximity between the client device 2B and the intraoral scanning device 10 within the threshold proximity, the wireless connection between the client device and the intraoral scanning device is then established 23. Cause, based on the detection of the wireless connection 23, to visually display 54 a wireless connection status of the wireless connection 21 of the intraoral scanning device 10 to the client device 2B on a graphical user interface.

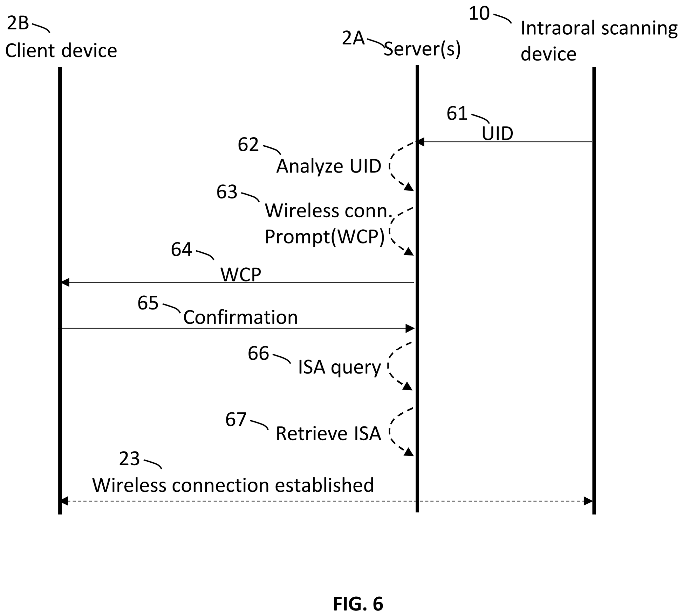

FIG. 6 illustrates and example where a wireless connection is established 23 between the client device 2B and the intraoral scanning device 10. In this example, the one or more servers is configured to determine 62 whether the intraoral scanning device is approved to establish the wireless connection with the client device based on the unique identifier 61 and generate a wireless connection prompt 63, in response to determining that the intraoral scanning device 10 is approved for wireless connecting with the client device 2B, and forward the wireless connection prompt to the client device 25. The one or more servers 2A is configured to determine, upon the client device 2B received the wireless connection prompt, whether a wireless connection confirmation 65 is received from the client device 2B, generate 66, in response to receiving the wireless connection confirmation 65, an intraoral scanning application query corresponding to the intraoral scanning device based at least in part on the unique identifier, query an intraoral scanning application server of the one or more servers with an intraoral scanning device application query to retrieve 67 an intraoral scanning application corresponding to the intraoral scanning device 10 from the intraoral scanning application server 2A, and establish the wireless connection 23 between the client device 2B and the intraoral scanning device 10.

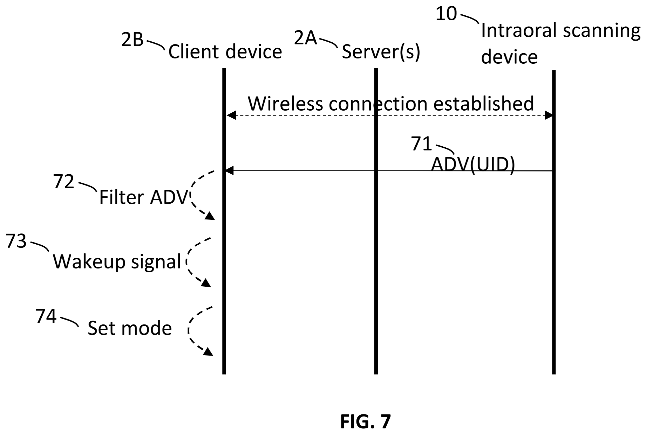

FIG. 7 illustrates an example where the client device is set into different power modes based on a wakeup signal. In this specific example, the client device 2B includes an antenna, an interface circuit, electrically coupled to the antenna, configured to communicate with the one or more servers and the intraoral scanning device, wherein the interface circuit is further configured to receive a beacon with advertising information 71 from the intraoral scanning device 10, and wherein the client device is configured to filter 72 the advertising information, determine that the filtered advertising information has changed relative to a previous version of the advertising information for the intraoral scanning device 10 received by the client device 2B, and provide 73 a wakeup signal in response to determining that the filtered advertising information has changed relative to a previous version of the advertising information, and set 74 the client device 2B from a power-saving mode to a normal operating mode based on the wakeup signal.

Although some embodiments have been described and shown in detail, the disclosure is not restricted to such details, but may also be embodied in other ways within the scope of the subject matter defined in the following claims. In particular, it is to be understood that other embodiments may be utilized, and structural and functional modifications may be made without departing from the scope of the present invention.

Benefits, other advantages, and solutions to problems have been described herein with regard to specific embodiments. However, the benefits, advantages, solutions to problems, and any component(s)/ unit(s) that may cause any benefit, advantage, or solution to occur or become more pronounced are not to be construed as critical, required, or essential features or components/ elements of any or all the claims or the invention. The scope of the invention is accordingly to be limited by nothing other than the appended claims, in which reference to a component/ unit/ element in the singular is not intended to mean "one and only one" unless explicitly so stated, but rather "one or more." A claim may refer to any of the preceding claims, and "any" is understood to mean "any one or more" of the preceding claims.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e., to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The step of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

### Items

1. An intraoral scanning system comprising:
   - an intraoral scanning device configured to acquire light information from reflections of a dental object during a scanning session,
   - one or more processors operably connected to the intraoral scanning device, and at least one of the one or more processors includes a client device and one or more servers, the one or more processors are configured to:
      ∘ determine surface information from the light information, and
      ∘ generate a three-dimensional (3D) surface model of the dental object using the surface information,
   - a wireless communication interface configured to transmit via a first wireless communication protocol a unique identifier of the intraoral scanning device, and wherein the client device is configured to receive the unique identifier via the first wireless communication protocol, and a wireless connection is established between the client device and the intraoral scanning device via the one or more servers upon a confirmation of the unique identifier by the client device.
2. The intraoral scanning system according to item 1, wherein the confirmation of the unique identifier is determined upon a user input, and the user input is a selection of the unique identifier.
3. The intraoral scanning system according to any of the previous items, wherein the first wireless communication protocol is based on a wireless proximity protocol, and the confirmation of the unique identifier is forwarded to the one or more servers, and the one or more servers is configured to establish the wireless connection between the client device and the intraoral scanning device via the one or more servers.
4. The intraoral scanning system according to any of the previous items, wherein the wireless connection includes at least a second wireless communication protocol that is different from the first wireless communication protocol.
5. The intraoral scanning system according to any of the previous items, wherein the one or more servers is configured to receive and forward the unique identifier to the client device via the first wireless communication protocol.
6. The intraoral scanning system according to any of the previous items, wherein the wireless communication interface is configured to transmit via the first wireless communication protocol a unique identifier upon no requests to the client device or the one or more servers.
7. The intraoral scanning system according to any of the previous items, wherein the surface information is transmitted, via the wireless connection, from the intraoral scanning device to the one or more servers, and the one or more servers is configured to generate the 3D surface model of the dental object using the surface information, and the 3D surface model is forwarded to the client device.
8. The intraoral scanning system according to item 7, comprising a displaying unit configured to be operably connected to the client device, and wherein the displaying unit is configured to display the 3D surface model.
9. The intraoral scanning system according to item 8, wherein the displaying unit is part of the client device.
10. The intraoral scanning system according to any of the previous items, wherein the first wireless communication protocol is configured to Wireless Fidelity (WIFI), 60-GHz wireless communication protocol, Bluetooth, Bluetooth low energy or Near-Field-Communication (NFC).
11. The intraoral scanning system according to item 4, wherein the second wireless communication protocol is configured to WIFI or 60-GHz wireless communication protocol.
12. The intraoral scanning system according to any of the previous items, wherein the intraoral scanning device transmits via the first wireless communication protocol proximity data that includes the unique identifier, and wherein the client device or the one or more servers is configured to:
   - analyse proximity data of the intraoral scanning device,
   - determine, responsive to the analysis, that a spatial proximity between the client device and the intraoral scanning device is within a threshold proximity;
   - detect, responsive to the detection of an execution of an application on the client device having the spatial proximity between the client device and the intraoral scanning device within the threshold proximity, the wireless connection between the client device and the intraoral scanning device; and
   - cause, based on the detection of the wireless connection, to visually display a wireless connection status of the wireless connection of the intraoral scanning device to the client device on a graphical user interface.
13. The intraoral scanning system according to item 12, wherein the wireless connection status includes a connection mode and an unconnected mode.
14. The intraoral scanning system according to any of items 12 or 13, comprising a non-transitory memory coupled to the one or more processors, the non-transitory memory including a set of instructions of computer-executable program code, which when executed by the one or more processors, cause the client device to concurrently detect a status of the intraoral scanning device and/or a status of the wireless connection between the client device and the intraoral scanning device.
15. The intraoral scanning system according to any of the previous items, wherein the one or more servers is configured to:
   - determine whether the intraoral scanning device is approved to establish the wireless connection with the client device based on the unique identifier,
   - generate a wireless connection prompt, in response to determining that the intraoral scanning device is approved for wireless connecting with the client device, and forward the wireless connection prompt to the client device,
   - determine, upon the client device received the wireless connection prompt, whether a wireless connection confirmation is received from the client device,
   - generate, in response to receiving the wireless connection confirmation, an intraoral scanning application query corresponding to the intraoral scanning device based at least in part on the unique identifier,
   - query an intraoral scanning application server of the one or more servers with an intraoral scanning device application query to retrieve an intraoral scanning application corresponding to the intraoral scanning device from the intraoral scanning application server, and
   - establish the wireless connection between the client device and the intraoral scanning device.
16. The intraoral scanning system according to any of the previous items, wherein the intraoral scanning device is configured to broadcast, by the wireless communication interface, a unique identifier of the intraoral scanning device via the first wireless communication protocol, and wherein the client device confirms the unique identifier and, the confirmation is forwarded to the one or more servers which then establish the wireless connection between the intraoral scanning device and the client device.
17. The intraoral scanning device according to item 7, wherein the wireless connection includes a second wireless communication protocol, wherein the surface information is transmitted via the second wireless communication protocol.
18. The intraoral scanning device according to item 17, wherein the second wireless communication protocol is an unsecured wireless network protocol.
19. The intraoral scanning system according to item 18, wherein the unsecured wireless network protocol is a User Datagram Protocol (UDP) or a Transmission Control Protocol (TCP).
20. The intraoral scanning device according to item 7, wherein the wireless connection includes a third wireless communication protocol, wherein control data of the intraoral scanning device or internal information of a dental object is transmitted via the third wireless communication protocol, and the third wireless communication protocol is a secured wireless network protocol.
21. The intraoral scanning system according to item 20, wherein the unsecured wireless network protocol is a User Datagram Protocol (UDP) or a Transmission Control Protocol (TCP).
22. The intraoral scanning system according to any of the previous items, wherein the client device includes:
   - an antenna,
   - an interface circuit, electrically coupled to the antenna, configured to communicate with the one or more servers and the intraoral scanning device, wherein the interface circuit is further configured to receive a beacon with advertising information for the intraoral scanning device, and wherein the client device is configured to:
      ∘ filter the advertising information,
      ∘ determine that the filtered advertising information has changed relative to a previous version of the advertising information for the intraoral scanning device received by the client device, and
      ∘ provide a wakeup signal in response to determining that the filtered advertising information has changed relative to a previous version of the advertising information, and
      ∘ set the client device from a power-saving mode to a normal operating mode based on the wakeup signal.
23. The intraoral scanning system according to item 22, wherein the advertising information includes the unique identifier.
24. The intraoral scanning system according to item 22, wherein the client device includes:
   - a processor of the one or more processors,
   - a memory, coupled to the processor, which stores a program module configured to be executed by the processor, the program module including:
   - instructions for receiving the beacon;
   - instructions for determining if the filtered advertising information is changed; and
   - instructions for providing the wakeup signal.
25. The intraoral scanning system according to any of the items, comprising a displaying unit configured to display a plurality of unique identifiers including the unique identifier and other unique identifiers of other intraoral scanning devices, and wherein the plurality of unique identifiers is listed according to a signal strength of a first wireless communication protocol associated to each of the plurality of unique identifiers.
26. The intraoral scanning system according to any of the items, wherein the intraoral scanning device is configured to generate a public-key based on the unique identifier, and the one or more processors is configured to:
   - receive the public key via the first wireless communication protocol,
   - generate a certificate based on the public key,
   - sign the certificate and transmit the signed certificate to the intraoral scanning device via the first wireless communication protocol, and
   wherein the intraoral scanning device is configured to generate a signature based on the signed certificate and transmit the signature via the first wireless communication protocol to the client device, and the client device is configured to verify the signature based on a public-key algorithm and a copy of the public-key, and the wireless connection is established when the signature is successfully verified by the client device.

## Claims

1. An intraoral scanning system comprising:
• an intraoral scanning device configured to acquire light information reflected from a dental object during a scanning session,
• one or more processors operably connected to the intraoral scanning device, and at least one of the one or more processors includes a client device and one or more servers, the one or more processors are configured to:
∘ determine surface information from the light information, and
∘ generate a three-dimensional (3D) surface model of the dental object using the surface information,
• a wireless communication interface configured to transmit via a first wireless communication protocol a unique identifier of the intraoral scanning device, and wherein the client device is configured to receive the unique identifier via the first wireless communication protocol, and a wireless connection is established between the client device and the intraoral scanning device via the one or more servers upon a confirmation of the unique identifier by the client device, and
wherein the wireless connection includes at least a second wireless communication protocol that is different from the first wireless communication protocol, and wherein the first wireless communication protocol includes a first security level and the second wireless communication protocol includes a second security level.

2. The intraoral scanning system according to claim 1, wherein the confirmation of the unique identifier is determined upon a user input, and the user input is a selection of the unique identifier.

3. The intraoral scanning system according to any of the previous claims, wherein the first wireless communication protocol is based on a wireless proximity protocol, and the confirmation of the unique identifier is forwarded to the one or more servers, and the one or more servers is configured to establish the wireless connection between the client device and the intraoral scanning device via the one or more servers.

4. The intraoral scanning system according to any of the previous claims, wherein the one or more servers is configured to receive and forward the unique identifier to the client device via the first wireless communication protocol.

5. The intraoral scanning system according to any of the previous claims, wherein the wireless communication interface is configured to transmit via the first wireless communication protocol a unique identifier upon no requests to the client device or the one or more servers.

6. The intraoral scanning system according to any of the previous claims, wherein the surface information is transmitted, via the wireless connection, from the intraoral scanning device to the one or more processors, and the one or more processors is configured to generate the 3D surface model of the dental object using the surface information, and the 3D surface model is forwarded to the client device.

7. The intraoral scanning system according to any of the previous claims, wherein the intraoral scanning device transmits via the first wireless communication protocol proximity data that includes the unique identifier, and wherein the client device or the one or more servers is configured to:
• analyse proximity data of the intraoral scanning device,
• determine, responsive to the analysis, that a spatial proximity between the client device and the intraoral scanning device is within a threshold proximity;
• detect, responsive to the detection of an execution of an application on the client device having the spatial proximity between the client device and the intraoral scanning device within the threshold proximity, the wireless connection between the client device and the intraoral scanning device; and
• cause, based on the detection of the wireless connection, to visually display a wireless connection status of the wireless connection of the intraoral scanning device to the client device on a graphical user interface.

8. The intraoral scanning system according to claim 7, wherein the wireless connection status includes a connection mode and an unconnected mode.

9. The intraoral scanning system according to any of the previous claims, wherein the one or more servers is configured to:
• determine whether the intraoral scanning device is approved to establish the wireless connection with the client device based on the unique identifier,
• generate a wireless connection prompt, in response to determining that the intraoral scanning device is approved for wireless connecting with the client device, and forward the wireless connection prompt to the client device,
• determine, upon the client device received the wireless connection prompt, whether a wireless connection confirmation is received from the client device,
• generate, in response to receiving the wireless connection confirmation, an intraoral scanning application query corresponding to the intraoral scanning device based at least in part on the unique identifier,
• query an intraoral scanning application server of the one or more servers with an intraoral scanning device application query to retrieve an intraoral scanning application corresponding to the intraoral scanning device from the intraoral scanning application server, and
• establish the wireless connection between the client device and the intraoral scanning device.

10. The intraoral scanning system according to any of the previous claims, wherein the intraoral scanning device is configured to broadcast, by the wireless communication interface, a unique identifier of the intraoral scanning device via the first wireless communication protocol, and wherein the client device confirms the unique identifier and, the confirmation is forwarded to the one or more servers which then establish the wireless connection between the intraoral scanning device and the client device.

11. The intraoral scanning device according to claim 6, wherein the wireless connection includes a second wireless communication protocol, wherein the surface information is transmitted via the second wireless communication protocol.

12. The intraoral scanning device according to claim 11, wherein the second wireless communication protocol is an unsecured wireless network protocol.

13. The intraoral scanning system according to claim 12, wherein the unsecured wireless network protocol is a User Datagram Protocol (UDP) or a Transmission Control Protocol (TCP).

14. The intraoral scanning device according to claim 6, wherein the wireless connection includes a third wireless communication protocol, wherein control data of the intraoral scanning device or internal information of a dental object is transmitted via the third wireless communication protocol, and the third wireless communication protocol is a secured wireless network protocol.

15. The intraoral scanning device according to any of the previous claims, wherein the first security level and the second security level are dynamically changing in a level of security depending on a connection quality between the intraoral scanning device and the client device.
